# EUROPEAN PATENT APPLICATION

(11) **EP 2 030 645 A1**
(43) Date of publication of application: **04.03.2009**
(21) Application number: 07115396.9
(22) Date of filing: 31.08.2007
(51) Int. Cl.: A61M 15/00

(54) **Dry-powder inhaler for treating respiratory diseases**

(71) Applicant: L'AIR LIQUIDE, Société Anonyme pour l'Etude et l'Exploitation des Procédés Georges Claude, 75007 Paris (FR)
(72) Inventor: Haussermann, Sabine, 78000, VERSAILLES (FR); Birngruber, Reginald, 23564, LUBECK (DE); Texereau, Joelle, 75013, PARIS (FR); Apiou, Gabriela, 75014, PARIS (FR)
(74) Representative: Pittis, Olivier

(57) **Abstract**

The invention concerns a dry powder drug inhaler (6) adapted for the administration of a dry powder drug to a patient comprising a housing (1) with a first compartment (4) for receiving a dry-powder drug (8) and a second compartment (2) for receiving an underpressure gas cartridge (7), the first compartment (4) comprising an outlet (10) for the delivering of a gaseous flow containing the dry-powder drug and the pressurized gas to a user ; gas passage means (9) for allowing a fluidic passage of pressurized gas from the second compartment (2) to the first compartment (4) ; and an actuating device (1), adapted to be actuated by the user, for releasing gas in the gas passage means (9) and in the first compartment (4) in response of their actuation by said user thereby obtaining the gaseous flow containing the dry-powder drug and the pressurized gas to a user. Preferably, the pressurized gas is a helium/oxygen mixture.

## Description

The invention concerns a dry-powder drug inhaler useable for treating various respiratory diseases.

Dry powder inhalers (DPI) are commonly used for treating respiratory diseases. The DPI contain the drug to be inhaled, which is in the form of a dry powder.

Said powder is normally dispersed into an aerosol by the inhalation force of the patient, when the patient is inhaling, i.e. during the inhalation phases of the patient.

The problem is that the dose depends on the inhalation force of the patient and can be therefore variable.

This could lead to a very bad deposition of the drug particles in the airways of the patient and thus to a low efficiency of the treatment.

Further, a high deposition of drug particles in the oropharynx will have no effect for treating the lung disease or delivering it systemically, but will only cause side effects.

Besides, a known inhaler, commonly used for administrating insulin by inhalation, disperses the powder with air under pressure in lieu of the natural flow of air created by the patient's breath, as air is the more common carrier for aerosols.

However, such a device is not ideal as compressed air exhibits low deposition properties, especially in the deep lung and this leads to a low uptake of insulin into the blood circulation.

A goal of the invention is hence to propose a dry powder inhaler overcoming the problems of the prior art, in particular a device that would work independently of the inhalation force of the patient, thereby improving the particle deposition and efficiency in the patient's airways.

Another goal of the invention is to propose a dry powder inhaler that can be used with gases other than air as other gases could have synergistic effects with the dispersed drugs and their use as carrier gases can be advantageous.

A solution according to the present invention is a dry powder drug inhaler adapted for the administration of a dry powder drug to a patient comprising a housing with:
- a first compartment for receiving a dry-powder drug and a second compartment for receiving an under-pressure gas cartridge, the first compartment comprising an outlet for the delivering of a gaseous flow containing the dry-powder drug and the pressurized gas to a user,
- gas passage means for allowing a fluidic passage of pressurized gas from the second compartment to the first compartment,
- an actuating device, adapted to be actuated by the user, for releasing gas in the gas passage means and in the first compartment in response of its actuation by said user thereby obtaining the gaseous flow containing the dry-powder drug and the pressurized gas to a user.

The dry powder drug inhaler can further comprise one or several of the additional following features :
- a gas-cartridge is arranged in the second compartment.
- the cartridge contains a helium/oxygen gas mixture.
- sealing means, like a gasket or similar, for example an O-ring, are arranged between said first compartment and second compartment.
- the actuating device is adapted to be actuated directly or indirectly by the user, in particular the actuating device is manually actuated by the user himself or automatically by means of user's inspiration or expiration phase detection sensor.
- an under-pressure gas cartridge is arranged in the second compartment.
- the second compartment comprises cartridge-connection means for connecting the cartridge and allowing a fluid gas communication between said cartridge and the first compartment and thereby allowing the passage of the gas from the cartridge to said first compartment comprising the powder drug.
- it comprises gas-regulation means adapted to reduce the pressure of the gas delivered by the cartridge.
- the outlet of the first compartment comes into a spacer or respiratory mask.
- the gas cartridge is a gas cylinder having a length of less than 20 cm, preferably less than 10 cm.
- it has total weight of less than 1 kg, preferably less than 0,5 kg.

Examples of devices according to the invention are represented on the enclosed Figures among which :
- Figure 1 represents a first embodiment of a dry powder inhaler, and
- Figure 2 represents a second embodiment of a dry powder inhaler equipped with a spacer.

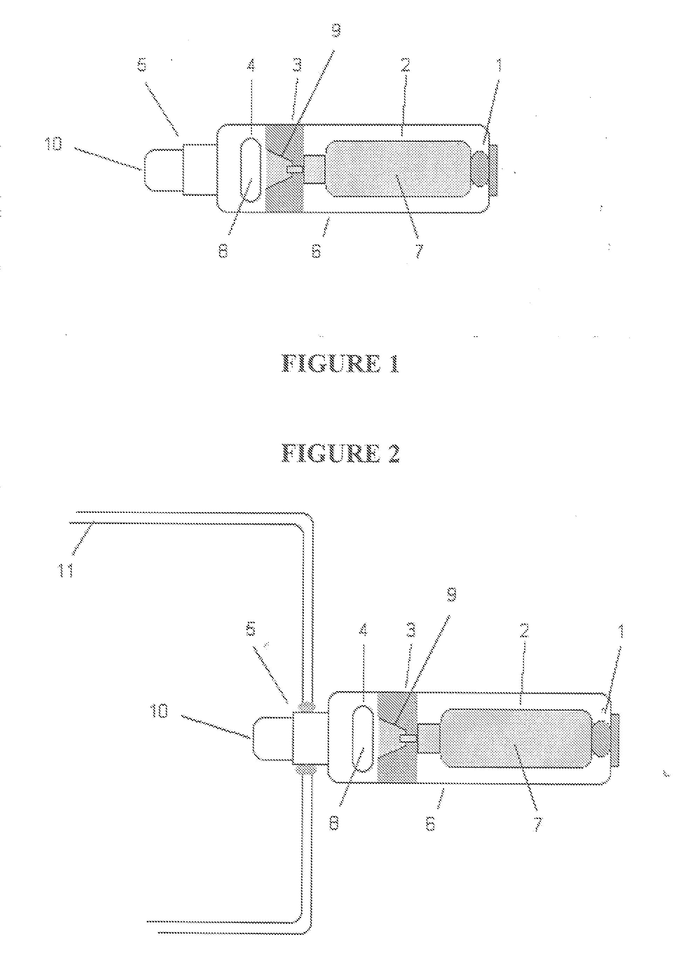

Figure 1 is a schematic view of a dry-powder drug inhaler 6 according to the present invention, which is adapted for the administration of a dry powder drug to a patient and which comprises a housing 1 or main body with two internal compartments 4,2.

The first compartment 4 is adapted to receive a dry-powder drug 8 that has to be administrated by the inhalation route to the patient. The dry-powder drug 8 is preferably conditioned in a small powder cartridge that is inserted into the first compartment 4 and that can be removed once empty and replaced by a new one.

The second compartment 2 is adapted for receiving an under-pressure gas cartridge 7 that is used for propelling and/or carrying the powder drug to the patient airways.

One idea of the invention is mainly to use a compressed gas, in particular helium or a helium/oxygen mixture, to disperse the powder, independently of the inhalation flow of the patient and hence improve deposition in the small airways, in healthy and compromised lung morphologies.

Gas mixtures such as He/O₂ mixtures are preferred as such mixtures have the ability of improving the particle deposition in the deep lung and therefore the uptake of the drug, for example insulin, into the blood circulation.

Further, the first compartment 4 further comprising an outlet 10 for delivering the gaseous flow containing the dry-powder drug and the pressurized gas via a mouthpiece to the patient,

The communication between both compartments 4, 2 is obtained thanks to gas passage means 9, such as an opening or a gas conduit arranged in the wall separating both compartments, that allow a fluidic passage of the pressurized gas from the gas cartridge 7 inserted in the second compartment 4 to the first compartment 2.

An actuating device 1 or means, adapted to be actuated by the user, such as a release push button or similar, is arranged on the dry powder drug inhaler 6 for controlling the release of gas in the gas passage means 9 and hence in the first compartment 2, thereby propelling and/or carrying the drug powder, in response of their actuation by the patient.

The actuating device 1 can also be actuated automatically, for example by a sensor that detects any inspiration phase of the patient and, in response to such a detection, liberate the carrier gas in the first compartment 4 containing the powder drug.

The gas capsule 7 can be opened to the inhaler reversibly or irreversibly. In the latter case, the gas capsule 7 is disposable. For instance, an helium/O₂ gaseous mixture can be provided to the inhaler 6 by a small and highly compressed gas capsule 7 or cartridge, in which the compressed gas mixture is conditioned and afterwards released.

The gas cartridge 7 preferably contain either compressed He or a He/O₂ mixture or a "premix" formed of compressed He or He/O₂ together with a liquid or a dry medication that has to be mixed with and/or inhaled together with the powder drug 8.

The high pressure gas in the capsule can disperse powder from a reservoir or powder from a capsule. Also, micronised powder with and without lactose can be used or porous particles.

The dry powder inhaler 6 of the invention leads to a gas-driven dispersion of the powder drug 8 contained in the first compartment.

Further, the dry powder inhaler 6 of the invention comprises a sealing gasket 3 that is used for sealing the connection between the cartridge containing the gas and the drug and a mouth piece 5, in which arrives the outlet 10 of the first compartment 4, for delivering the gaseous product, i.e. drug and gas, to the patient.

Figure 2 represents a second embodiment of a device according to Figure 1, but also comprising a spacer 11 that is used for drug inhalation by the patient.

A spacer is a well-known kind of inhalation device comprising a main hollow body through which circulates the gas flow that has to be inhaled by the patient, which hollow body comprises, at one end, an opening in fluid communication with an inhalation mask or mouthpiece. The mask or the spacer 11 can be inseparable attached to the inhaler 6 or attached by inserting the mouthpiece 10 of the inhaler into a sealing gasket. An example of spacer is disclosed in document EP-A-514085.

Here again, the dispersion of the powder 4 takes place by the gas 2, which is actuated by the button 1 on the back of the device or by triggering with the inhalation of the patient by means of a sensor (not shown) that detects the inspiration phases of the patient, i.e. the inhalation and/or expiration phases.

The advantage of the device of the invention comprising the two compartments 4, 2 is, that the dispersion of the powder 8 is done by the force of the pressurized gas delivered by the cartridge 7 and not by the inhalation force of the patient. Also, using preferably an helium/oxygen mixture will increase the peripheral deposition of the drug and encourage homogeneous deposition throughout the lungs, especially a helium/oxygen mixture containing from 60/40 to 80/20 vol. % of helium, as such a mixture increases peripheral deposition. Such a gas mixture is disclosed in document EP-A-1064945.

The invention also concerns a method for administrating a dry powder drug to a patient comprising the steps of providing a patient having a respiratory disease and administrating to the airways of said patient, a dry powder drug efficient against said respiratory disease by means of a dry powder drug inhaler according to the present invention.

The device of the invention car be used in various medical applications, for instance to administrate anti-inflammatory drugs, bronchodilators, anti-tumoral agents, anti-viral drugs or antibiotics for respiratory diseases, but also systemic drugs delivered by the inhaled route, like insulin or similar.

## Claims

1. Dry powder drug inhaler (6) adapted for the administration of a dry powder drug to a patient comprising a housing (1) with:
- a first compartment (4) for receiving a dry-powder drug (8) and a second compartment (2) for receiving an under-pressure gas cartridge (7), the first compartment (4) comprising an outlet (10) for the delivering of a gaseous flow containing the dry-powder drug and the pressurized gas to a user,
- gas passage means (9) for allowing a fluidic passage of pressurized gas from the second compartment (2) to the first compartment (4),
- an actuating device (1), adapted to be actuated by the user, for releasing gas in the gas passage means (9) and in the first compartment (4) in response of its actuation by said user thereby obtaining the gaseous flow containing the dry-powder drug and the pressurized gas to a user.

2. Inhaler according to Claim 1, **characterized in that** a gas-cartridge (7) is arranged in the second compartment (2).

3. Inhaler according to any one of Claims 1 or 2, **characterized in that** sealing means (3) are arranged between said first compartment (4) and second compartment (2).

4. Inhaler according to any one of Claims 1 to 3, **characterized in that** the actuating device (1) is adapted to be actuated directly or indirectly by the user, in particular the actuating device (1) are manually actuated by the user himself or automatically by means of user's inspiration or expiration phase detection sensor.

5. Inhaler according to any one of Claims 1 to 4, **characterized in that** an under-pressure gas cartridge (7) is arranged in the second compartment (2).

6. Inhaler according to any one of Claims 1 to 5, **characterized in that** the second compartment (2) comprises cartridge-connection means for connecting the cartridge (7) and allowing a fluid gas communication between said cartridge (7) and the first compartment (4) and thereby allowing the passage of the gas from the cartridge (7) to said first compartment (4) comprising the powder drug (4).

7. Inhaler according to any one of Claims 1 to 6, **characterized in that** it comprises gas-regulation means adapted to reduce the pressure of the gas delivered by the cartridge (7).

8. Inhaler according to any one of Claims 1 to 7, **characterized in that** the outlet (10) of the first compartment (2) comes into a spacer (11) or respiratory mask.

9. Inhaler according to any one of Claims 1 to 8, **characterized in that** the gas cartridge (7) is a gas cylinder having a length of less than 20 cm, preferably less than 10 cm and/or it has total weight of less than 1 kg, preferably less than 0,5 kg.

10. Inhaler according to any one of Claims 1 to 9, **characterized in that** the gas-cartridge (7) arranged in the second compartment (2) contains a helium/oxygen gas mixture.
